(19) **Europäisches Patentamt / European Patent Office / Office européen des brevets**

(11) **EP 2 979 756 A1**

(12) **EUROPEAN PATENT APPLICATION**
published in accordance with Art. 153(4) EPC

(43) Date of publication:
**03.02.2016 Bulletin 2016/05**

(51) Int Cl.:
**B01J 23/63** (2006.01)    **C01B 3/02** (2006.01)
**H01M 8/06** (2006.01)

(21) Application number: **14775297.6**

(22) Date of filing: **25.03.2014**

(86) International application number:
**PCT/JP2014/058281**

(87) International publication number:
**WO 2014/157202 (02.10.2014 Gazette 2014/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **28.03.2013 JP 2013068638**

(71) Applicant: **JX Nippon Oil & Energy Corporation**
**Chiyoda-ku**
**Tokyo 100-8162 (JP)**

(72) Inventors:
- **NAKAIZUMI, Tomomi**
  **Tokyo 100-8162 (JP)**
- **FURUTA, Satoshi**
  **Tokyo 100-8162 (JP)**

(74) Representative: **Grünecker Patent- und Rechtsanwälte**
**PartG mbB**
**Leopoldstraße 4**
**80802 München (DE)**

(54) **DEHYDROGENATION CATALYST FOR NAPHTHENIC HYDROCARBONS, METHOD FOR PRODUCING DEHYDROGENATION CATALYST FOR NAPHTHENIC HYDROCARBONS, SYSTEM FOR PRODUCING HYDROGEN, AND METHOD FOR PRODUCING HYDROGEN**

(57)    A dehydrogenation catalyst for naphthenic hydrocarbons excellent in dehydrogenation activity is provided. One aspect of a dehydrogenation catalyst for naphthenic hydrocarbons according to the present invention comprises a carrier containing aluminum oxide; platinum; and a group 3 metal.

Fig.1

**Description**

**Technical Field**

The present invention relates to a dehydrogenation catalyst for naphthenic hydrocarbons, a method for producing the dehydrogenation catalyst, and a system for producing hydrogen and a method for producing hydrogen using the dehydrogenation catalyst.

**Background Art**

In recent years, it has been expected that fuel cells using hydrogen having a small environmental load as a fuel are used for the power sources of automobiles and the like. In the processes of the transport, storage, and supply of hydrogen, a naphthenic hydrocarbon (cyclic hydrocarbon) is used. For example, in a facility for producing hydrogen, a naphthenic hydrocarbon is produced by the hydrogenation of an aromatic hydrocarbon. This naphthenic hydrocarbon is transported to a hydrogen-consuming area or stored in a consuming area. In the consuming area, hydrogen and an aromatic hydrocarbon are produced by the dehydrogenation of the naphthenic hydrocarbon. This hydrogen is supplied to fuel cells. Naphthenic hydrocarbons are liquid at normal temperature, have a smaller volume than a hydrogen gas, and have lower reactivity and are safer than a hydrogen gas. Therefore, naphthenic hydrocarbons are more suitable for transport and storage than a hydrogen gas.

As a dehydrogenation catalyst for naphthenic hydrocarbons, a catalyst in which a bimetal of platinum-rhenium is supported on an alumina carrier is known (see the following Non Patent Literature 1).

**Citation List**

**Non Patent Literature**

Non Patent Literature 1: R. W. Coughlin, K. Kawakami, Akram Hasan, Journal of Catalysis, Vol. 88, 150-162 (1984).

**Summary of Invention**

**Technical Problem**

It is an object of the present invention to provide a dehydrogenation catalyst for naphthenic hydrocarbons excellent in dehydrogenation activity, a method for producing the dehydrogenation catalyst, and a system for producing hydrogen and a method for producing hydrogen using the dehydrogenation catalyst.

**Solution to Problem**

A dehydrogenation catalyst for naphthenic hydrocarbons according to one aspect of the present invention comprises a carrier containing aluminum oxide; platinum; and a group 3 metal.
In the above dehydrogenation catalyst according to one aspect of the present invention, an amount of the group 3 metal supported may be 0.1 to 5.0% by mass in terms of an oxide of the group 3 metal based on a total mass of the aluminum oxide.
In the above dehydrogenation catalyst according to one aspect of the present invention, when an amount of the platinum supported is $m_P$% by mass in terms of a platinum elemental substance based on the total mass of the aluminum oxide, and the amount of the group 3 metal supported is $m_3$% by mass in terms of the oxide of the group 3 metal based on the total mass of the aluminum oxide, $m_3/m_P$ may be (10/3) to 4.
A method for producing a dehydrogenation catalyst for naphthenic hydrocarbons according to one aspect of the present invention comprises a step of making a carrier which contains aluminum oxide and on which a group 3 metal is supported; and a step of supporting a solution of a platinum compound on the carrier and calcining the carrier.
In the above method for producing a dehydrogenation catalyst according to one aspect of the present invention, the platinum compound may contain an amine or ammonia.
In the above method for producing a dehydrogenation catalyst according to one aspect of the present invention, the carrier may be made by kneading a hydroxide of aluminum in a pseudoboehmite state, an aqueous solution of a nitrate of the group 3 metal, and nitric acid to prepare a kneaded material, making a pellet by extrusion of the kneaded material, and calcining the pellet.
A system for producing hydrogen according to one aspect of the present invention comprises a dehydrogenation reactor having the above dehydrogenation catalyst and producing hydrogen by dehydrogenation of a naphthenic hydrocarbon using the above dehydrogenation catalyst.

A method for producing hydrogen according to one aspect of the present invention comprises a step of producing hydrogen by dehydrogenation of a naphthenic hydrocarbon using the above dehydrogenation catalyst

**Advantageous Effects of Invention**

According to the present invention, a dehydrogenation catalyst for naphthenic hydrocarbons excellent in dehydrogenation activity, a method for producing the dehydrogenation catalyst, and a system for producing hydrogen and a method for producing hydrogen using the dehydrogenation catalyst can be provided.

**Brief Description of Drawings**

[Figure 1] Figure 1 is a diagram showing the relationship between the type of a group 3 metal that a dehydrogenation catalyst contains and the platinum surface area.

[Figure 2] Figure 2 is a diagram showing the relationship between the elapsed time of the dehydrogenation reaction of methylcyclohexane (MCH) using a dehydrogenation catalyst containing scandium and the conversion rate of MCH.

[Figure 3] Figure 3 is a diagram showing the relationship between the elapsed time of the dehydrogenation reaction of methylcyclohexane (MCH) using a dehydrogenation catalyst containing cerium and the conversion rate of MCH.

[Figure 4] Figure 4 is a diagram showing the relationship between the amount of cerium supported and the platinum surface area in dehydrogenation catalysts.

[Figure 5] Figure 5 is a schematic diagram showing one embodiment of a system for producing hydrogen according to the present invention.

[Figure 6] Figure 6 is a diagram showing the relationship between the amount of cerium oxide supported and the platinum surface area in dehydrogenation catalysts according to the present invention.

[Figure 7] Figure 7 is a diagram showing the relationship between the amount of platinum supported, the platinum dispersion degree, and the relative reaction rate of a dehydrogenation reaction in dehydrogenation catalysts according to the present invention.

[Figure 8] Figure 8 is a diagram showing the relationship between the molar ratio of a group 3 metal and platinum and the molar ratio of CO adsorbed on platinum in dehydrogenation catalysts according to the present invention.

**Description of Embodiments**

Preferred embodiments of the present invention will be described below. However, the present invention is not limited to the following embodiments in any way.

(Dehydrogenation Catalyst for Naphthenic Hydrocarbons)

A dehydrogenation catalyst for naphthenic hydrocarbons according to this embodiment comprises a carrier containing aluminum oxide, platinum (Pt), and a group 3 metal.

The platinum is supported on the carrier as a large number of atoms, clusters, or fine particles. In other words, the platinum is present on the surface of the aluminum oxide contained in the carrier. The surface is a portion where an active metal is substantially supported, and a catalytic reaction proceeds, and its thickness may be equal to or more than a single molecule of aluminum oxide. The particle diameter of the fine particles of platinum supported on the carrier is not particularly limited but may be, for example, 10 nm or less. The group 3 metal is also supported on the carrier like the platinum. In other words, the group 3 metal is present on the surface of the aluminum oxide. In addition, the group 3 metal may be contained within the aluminum oxide. In other words, it is possible that in the crystal structure of the aluminum oxide, part of the aluminum (Al) is replaced by the group 3 metal, and part of the carrier is an oxide of the group 3 metal (for example, cerium oxide).

When a naphthenic hydrocarbon comes into contact with the dehydrogenation catalyst in a reducing atmosphere, the platinum that is an active site abstracts at least a pair of hydrogen atoms from the naphthenic hydrocarbon, and a hydrogen molecule and an unsaturated hydrocarbon (for example, an aromatic hydrocarbon) are produced. The activity of the catalyst that promotes such a dehydrogenation reaction is referred to as dehydrogenation activity.

The dehydrogenation activity is evaluated, for example, based on the conversion rate C of the naphthenic hydrocarbon (unit: mol %) defined by the following numerical formula (1). The conversion rate C being high means that the dehydrogenation activity of the dehydrogenation catalyst is high.

$$\text{conversion rate } C = (M2/M1) \times 100 = \{M2/(M2 + M3)\} \times 100 \qquad (1)$$

wherein M1 is the number of moles of the naphthenic hydrocarbon supplied to a reaction container in which the dehydrogenation catalyst is placed. M2 is the number of moles of the aromatic hydrocarbon included in the products of the dehydrogenation reaction. M3 is the number of moles of the naphthenic hydrocarbon remaining after the dehydrogenation reaction. For example, when the conversion rate C is the conversion rate of methylcyclohexane, M1 and M3 are each the number of moles of methylcyclohexane, and M2 is the number of moles of toluene.

The present inventors consider that in the present invention, when cerium is added to aluminum oxide, due to its physical, electronic, or chemical action, platinum acting as an active site on the catalyst surface increases, or the function of platinum as an active site increases. In addition, the present inventors consider that one of more specific reasons why the effects of the present invention are achieved is as follows. However, the specific reasons why the effects of the present invention are achieved are not limited to the following.

In this embodiment, the group 3 metal adheres to the aluminum oxide or is contained within the aluminum oxide, and therefore part of the oxygen constituting the aluminum oxide or the oxide of the group 3 metal is eliminated from the carrier in a reducing atmosphere, and a large number of lattice defects are formed in the carrier. The platinum gets into these lattice defects, and thus the platinum is fixed to the carrier. As a result, the platinum reaches a more highly dispersed state by heating during the dehydrogenation reaction, and the movement and aggregation of the platinum on the carrier surface are suppressed. In other words, in this embodiment, compared with conventional dehydrogenation catalysts containing no group 3 metal, the platinum surface area during the dehydrogenation reaction increases, and the aggregation of the platinum is suppressed, and therefore the dehydrogenation activity and the durability improve. Such an effect is significant when the dehydrogenation catalyst contains cerium (Ce) as the group 3 metal. The formation of lattice defects due to the group 3 metal can be confirmed, for example, by observing the chemical shift of a peak derived from the group 3 metal constituting the oxide in the photoelectron spectroscopy (XPS) spectrum of the dehydrogenation catalyst.

In addition, in this embodiment, compared with dehydrogenation catalysts containing no group 3 metal, the aggregation of the platinum during the dehydrogenation reaction is suppressed, and therefore deactivation over time accompanying the dehydrogenation reaction is suppressed, and the dehydrogenation activity is easily maintained generally constant with the elapse of reaction time. On the other hand, the dehydrogenation activity of dehydrogenation catalysts containing no group 3 metal continues to decrease with the elapse of reaction time. In other words, in this embodiment, compared with dehydrogenation catalysts containing no group 3 metal, the catalyst life improves.

Further, in this embodiment, the dehydrogenation catalyst contains the group 3 metal, and thus its dehydrogenation activity improves, and therefore even if its amount of platinum supported is the same as that of conventional dehydrogenation catalysts containing no group 3 metal, it is possible to have better dehydrogenation activity than the conventional dehydrogenation catalysts. In other words, in this embodiment, even when its amount of platinum supported is smaller than that of conventional dehydrogenation catalysts containing no group 3 metal, it is possible to have dehydrogenation activity equal to that of the conventional dehydrogenation catalysts. Therefore, in this embodiment, it becomes possible to reduce the amount of expensive platinum supported without sacrificing dehydrogenation activity. The above effect due to the group 3 metal has been confirmed for the first time by the combination of aluminum oxide and platinum.

The group 3 metal increases the platinum surface area as described above, but the group 3 metal itself does not function as the active site of the dehydrogenation catalyst. Therefore, even if only the group 3 metal of the platinum and the group 3 metal is supported on the carrier, the catalyst cannot have high dehydrogenation activity.

As the naphthenic hydrocarbon (cyclic hydrocarbon), for example, one or more selected from the group consisting of cyclohexane, methylcyclohexane, dimethylcyclohexane, decalin, 1-methyldecalin, 2-methyldecalin, and 2-ethyldecalin may be used. These compounds are referred to as organic hydrides.

The surface of the carrier may be composed of porous aluminum oxide. The carrier has any of the following functions:

Amain catalyst is helped to increase its catalytic activity or selectivity.
The dispersibility of an active metal is increased.
The catalyst life is extended.
The mechanical strength as the structure of a catalyst is increased.
A catalyst is solidified.
The molding of a catalyst is made possible.
The carrier is a structure that substantially supports an active metal.

The type of the aluminum oxide is not limited, and specific examples of the aluminum oxide include $\alpha$-alumina, $\delta$-alumina, $\theta$-alumina, $\gamma$-alumina, or alumite. The specific surface area of the aluminum oxide is not particularly limited but falls within the range of approximately 100 to 500 $m^2/g$.

The shape of carrier is not particularly limited. For example, the carrier may be in the form of pellets, may be in the form of a plate, or may be in the form of a honeycomb.

As the group 3 metal, one or more selected from scandium (Sc), yttrium (Y), lanthanoids, and actinoids may be used. Examples of the lanthanoids include lanthanum (La) or cerium (Ce). Among these group 3 metals, cerium is most preferred. As described above, by containing cerium, the dehydrogenation catalyst can have significantly high dehydrogenation activity.

The amount of platinum supported in the dehydrogenation catalyst is not particularly limited and may be 0.1 to 5.0% by mass or 0.2 to 1.0% by mass in terms of a platinum elemental substance based on the total mass of the aluminum oxide. The amount of platinum supported is equal to or more than the above lower limit value, and thus the dehydrogenation activity improves more. When the amount of platinum supported is equal to or more than the above upper limit value, the extent of improvement in catalytic activity accompanying an increase in the amount of platinum supported becomes mild. In addition, the price of platinum is very high, and therefore the amount of platinum supported is limited for the practical use of the dehydrogenation catalyst. Even when the amount of platinum supported is outside the above numerical value range, the effects of the present invention are achieved.

The amount of the group 3 metal supported in the dehydrogenation catalyst is not particularly limited and may be 0.1 to 5.0% by mass in terms of the oxide of the group 3 metal based on the total mass of the aluminum oxide. The amount of the group 3 metal supported is equal to or more than the above lower limit value, and thus the platinum surface area increases more, and the dehydrogenation activity improves more. The amount of the group 3 metal supported is equal to or less than the above upper limit value, and thus the platinum surface area is easily increased while the mechanical strength of the dehydrogenation catalyst is maintained. In addition, the amount of the group 3 metal supported is equal to or less than the above upper limit value, and thus the molding of the carrier in the production process becomes easy. When the amount of the group 3 metal supported greatly exceeds the above upper limit value, there is a tendency that the performance of the carrier decreases, and the platinum surface area decreases. But, even when the amount of the group 3 metal supported is outside the above numerical value range, the effects of the present invention are achieved. For example, the amount of the group 3 metal supported in the dehydrogenation catalyst may be larger than 0% by mass and 20% by mass or less in terms of the oxide of the group 3 metal. The amount of the group 3 metal supported in the dehydrogenation catalyst may be larger than 0% by mass and 10% by mass or less in terms of the oxide of the group 3 metal. The amount of the group 3 metal supported in the dehydrogenation catalyst may be 0.3 to 5.0% by mass or 2.0 to 3.0% by mass in terms of the oxide of the group 3 metal. When the amount of the group 3 metal supported is 2.0 to 3.0% by mass in terms of the oxide of the group 3 metal, there is a tendency that the platinum surface area particularly increases easily.

When the amount of platinum supported is $m_P$% by mass in terms of a platinum elemental substance based on the total mass of the aluminum oxide, and the amount of the group 3 metal supported is $m_3$% by mass in terms of the oxide of the group 3 metal based on the total mass of the aluminum oxide, $m_3/m_P$ may be (10/3) to 4. In this case, there is a tendency that the platinum dispersion degree is high, and the reaction rate of the dehydrogenation reaction is high. For the reason that there is a tendency that the platinum dispersion degree is high, and the reaction rate of the dehydrogenation reaction is high, $m_3/m_P$ may be 2.78 to 3.64.

(Method for Producing Dehydrogenation Catalyst)

The dehydrogenation catalyst according to this embodiment is produced, for example, by a method comprising the step of supporting a group 3 metal and the step of supporting platinum following the step of supporting a group 3 metal as follows. In the step of supporting a group 3 metal, a carrier which contains aluminum oxide and on which a group 3 metal is supported is made. In the step of supporting platinum, a solution of a platinum compound is supported on the carrier, and the carrier is calcined.

[Step of Supporting Group 3 Metal]

A solution (for example, an aqueous solution) of a group 3 metal compound is supported on a carrier (for example, a porous aluminum oxide carrier). Examples of the supporting method include an incipient wetness method, a pore filling method, an adsorption method, an immersion method, an evaporation-to-dryness method, a spraying method, an ion exchange method, and a liquid phase reduction method. The salt of the group 3 metal is adhered to the surface of the carrier by these methods. The amount of the group 3 metal supported in the dehydrogenation catalyst may be adjusted by the concentration or amount of the group 3 metal compound.

As the group 3 metal compound, for example, nitrates, sulfates, carbonates, acetates, phosphates, oxalates, borates, chlorides, alkoxides, and acetylacetonates may be used.

By calcining the carrier to which the salt of the group 3 metal adheres to decompose the salt, the group 3 metal is supported on the carrier. The calcining temperature may be a temperature at which the thermal decomposition of the salt proceeds and may be, for example, about 300 to 600°C.

Instead of the above supporting step, there is also a method of mixing a group 3 metal compound into aluminum oxide or its precursor before having a stable porous structure, and forming a mixture having a porous structure. Examples of such a method include a kneading method, a sol-gel method, and a coprecipitation method. Alternatively, aluminum oxide and an oxide of a group 3 metal may be physically mixed. A group 3 metal may be added to a carrier by the step of molding a mixture of a powder of a raw material of a carrier and a group 3 metal compound and calcining the molded body. As the powder of a raw material of a carrier, for example, Boehmite, which is a raw material of $\gamma$-alumina, may be used. The calcining temperature in this case may be a temperature at which the thermal decomposition of the group 3 metal compound proceeds, and $\gamma$-alumina is produced by the sintering of boehmite. Such a calcining temperature is, for example, about 300 to 600°C.

A carrier may be made by kneading a hydroxide of aluminum in a pseudoboehmite state, an aqueous solution of a nitrate of a group 3 metal, and dilute nitric acid to prepare a kneaded material, making a pellet by the extrusion of the kneaded material, and calcining the pellet. By making a carrier by such a method, the group 3 metal disperses easily in the carrier. In a dehydrogenation catalyst made using such a carrier, the platinum surface area increases easily, and high dehydrogenation activity is easily obtained. The hydroxide of aluminum in a pseudoboehmite state is represented, for example, by the composition formula $AlOOH$ or $Al_2O_3 \cdot H_2O$. The kneaded material is also referred to as dough. The pH of the kneaded material may be adjusted to 3 to 7. By the adjustment of pH, the kneaded material has moderate viscosity, and the kneaded material is easily molded. The pH of the kneaded material fluctuates with the amount of nitric acid added. The pH of the kneaded material may be adjusted by adding ammonia water to the kneaded material.

[Step of Supporting Platinum]

A solution (for example, an aqueous solution) of a platinum compound is supported on the carrier on which the group 3 metal is supported. Examples of the supporting method include an incipient wetness method, a pore filling method, an adsorption method, an immersion method, an evaporation-to-dryness method, a spraying method, an ion exchange method, and a liquid phase reduction method. The platinum compound is adhered to the surface of the carrier by these methods. The amount of platinum supported in the dehydrogenation catalyst may be adjusted by the concentration or amount of the platinum compound.

The platinum compound is not particularly limited but is required to be soluble in liquid solvents. For example, tetrachloroplatinic acid, potassium tetrachloroplatinate, ammonium tetrachloroplatinate, sodium tetrachloroplatinate, bis(acetylacetonato)platinum, diamminedichloroplatinum, dinitrodiammineplatinum, dinitrodiammineplatinum nitrate, dinitrodiammineplatinum ammonia solutions, ethanolamineplatinum, tetraammineplatinum dichloride, tetraammineplatinum hydroxy salts, tetraammineplatinum nitrate, tetraammineplatinum acetate, tetraamminneplatinum carbonate, tetraammineplatinum phosphate, hexaammineplatinum tetrachloride, hexaammineplatinum hydroxy salts, bis(ethanolammonium)hexahydroxoplatinum(IV), sodium hexahydroxoplatinate(IV), potassium hexahydroxoplatinate(IV), platinum nitrate, and platinum sulfate may be used. It is preferred that the platinum compound contains an amine or ammonia. In this case, the platinum surface area in the dehydrogenation catalyst increases easily. For the platinum compound containing an amine or ammonia, when the platinum compound that may be at least one selected from the group consisting of dinitrodiammineplatinum nitrate, dinitrodiammineplatinum ammonia solutions, ethanolamineplatinum, and hexaammineplatinum hydroxy salts is dinitrodiammineplatinum nitrate or a dinitrodiammineplatinum ammonia solution, platinum disperses uniformly easily in the carrier, and the platinum surface area increases easily. When the platinum compound is ethanolamineplatinum, platinum is easily selectively distributed in the outer shell portion (the vicinity of the outer surface) of the carrier, and the platinum surface area increases easily.

By calcining the carrier on which the platinum compound is supported to decompose the platinum compound, platinum is supported on the carrier, and the dehydrogenation catalyst according to this embodiment is completed. The calcining temperature may be a temperature at which the decomposition of the platinum compound proceeds and may be, for example, about 200 to 500°C. Particularly by performing calcining at 350°C or less, the aggregation of platinum during calcining is less likely to occur, and the platinum surface area in the dehydrogenation catalyst increases easily.

As described above, when a group 3 metal is supported on a carrier, it is preferred to perform calcining at such a high temperature that a group 3 metal compound decomposes. On the other hand, when platinum is supported on the carrier, it is preferred to perform calcining at such a low temperature that the aggregation of platinum is less likely to occur. In order to achieve both these conflicting conditions, in this embodiment, a group 3 metal and platinum are not simultaneously supported on a carrier, and both metals are individually supported on a carrier in the above two steps in which the calcining temperature is different. Thus, a dehydrogenation catalyst excellent in dehydrogenation activity can be easily produced.

(System for Producing Hydrogen, and Method for Producing Hydrogen)

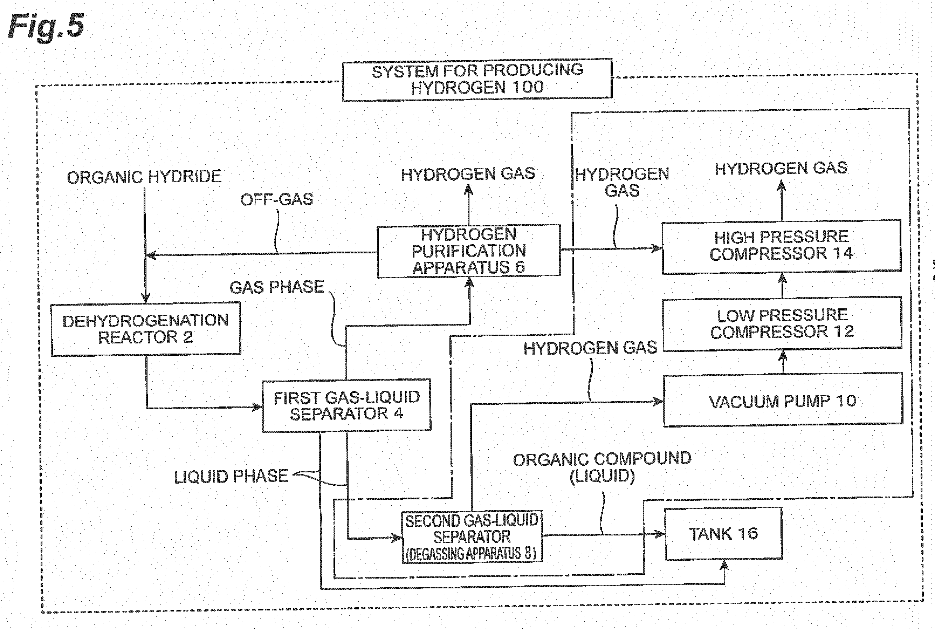

In this embodiment, hydrogen is produced using a system for producing hydrogen 100 shown in Figure 5. The system for producing hydrogen 100 is, for example, a hydrogen production system in a hydrogen station for supplying a hydrogen gas to a fuel cell vehicle as a fuel.

The system for producing hydrogen 100 according to this embodiment comprises at least a dehydrogenation reactor 2, a first gas-liquid separator 4, a hydrogen purification apparatus 6, and a tank 16. The dehydrogenation reactor 2 has the dehydrogenation catalyst according to this embodiment described above and produces hydrogen and an organic compound (an aromatic hydrocarbon or the like) by the dehydrogenation of a naphthenic hydrocarbon (organic hydride) using the dehydrogenation catalyst. In other words, a method for producing hydrogen according to this embodiment comprises the step of producing hydrogen and an organic compound by the dehydrogenation of a naphthenic hydrocarbon using the dehydrogenation catalyst according to this embodiment described above (dehydrogenation step).

In the dehydrogenation step, a naphthenic hydrocarbon is supplied into the dehydrogenation reactor 2. The dehydrogenation catalyst according to this embodiment described above is placed in the dehydrogenation reactor 2. The inside of the dehydrogenation reactor is a reducing atmosphere. When the naphthenic hydrocarbon comes into contact with the dehydrogenation catalyst in the dehydrogenation reactor 2, a dehydrogenation reaction occurs, and at least a pair of hydrogen atoms is abstracted from the naphthenic hydrocarbon, and a hydrogen molecule and an organic compound such as an aromatic hydrocarbon are produced. In this manner, the dehydrogenation reaction is a gas phase reaction.

The products of the dehydrogenation reaction (the hydrogen molecule and the organic compound) are supplied from the dehydrogenation reactor 2 into the first gas-liquid separator 4. The temperature in the first gas-liquid separator 4 is equal to or more than the melting point of the organic compound and less than the boiling point of the organic compound. The pressure in the first gas-liquid separator 4 is normal pressure (generally atmospheric pressure). Therefore, the hydrogen molecule in the first gas-liquid separator 4 is a gas, and the organic compound in the first gas-liquid separator 4 is a liquid. In other words, in the first gas-liquid separator 4, the products of the dehydrogenation reaction separate into a hydrogen gas (a gas phase, a gas layer) and a liquid of the organic compound (a liquid phase, a liquid layer). The gas phase (hydrogen-containing gas) in the first gas-liquid separator 4 is supplied to the hydrogen purification apparatus 6. The liquid phase (the liquid of the organic compound) in the first gas-liquid separator 4 is supplied to the tank 16. A vapor of the organic compound may be mixed in the gas phase. The partial pressure of the organic compound in the gas phase is about the saturated vapor pressure of the organic compound at the maximum. On the other hand, part of the hydrogen produced by dehydrogenation (a slight amount of a hydrogen gas) is dissolved in the liquid phase. In addition, the organic hydride not dehydrogenated may remain in the liquid phase.

The production system 100 may further comprise a second gas-liquid separator. The liquid phase (the liquid of the organic compound) in the first gas-liquid separator 4 may be supplied to the second gas-liquid separator instead of being supplied to a fuel cell vehicle. The second gas-liquid separator will be described as a degassing apparatus 8 below. The hydrogen gas dissolved in the liquid of the organic compound may be separated from the liquid using the degassing apparatus 8. The degassing apparatus 8 may comprise, for example, a separation membrane through which only the hydrogen gas of the hydrogen gas and the organic compound passes selectively. The hydrogen gas is separated from the organic compound using this separation membrane. The separation membrane may be, for example, a metal membrane (a PbAg-based membrane, a PdCu-based membrane, a Nb-based membrane, or the like), an inorganic membrane (a silica membrane, a zeolite membrane, a carbon membrane, or the like), or a polymer membrane (a fluororesin membrane, a polyimide membrane, or the like). The degassing apparatus 8 is not limited to an apparatus comprising a separation membrane. The degassing apparatus 8 may be an apparatus that carries out a method of changing gas solubility in a liquid by changing pressure or temperature, and performing degassing.

The hydrogen gas separated from the organic compound by the degassing apparatus 8 is supplied to a low pressure compressor 12 via a vacuum pump 10 and compressed. The hydrogen gas compressed in the low pressure compressor 12 is further compressed in a high pressure compressor 14 and then used as a fuel for fuel cells. On the other hand, the liquid of the organic compound separated from the hydrogen gas by the degassing apparatus 8 is supplied into the tank 16. The organic compound in the tank 16 may be reused as the organic hydride by being hydrogenated. The hydrogen gas is separated from the liquid phase (the liquid of the organic compound) by the above method. However, the system for producing hydrogen 100 need not comprise the degassing apparatus 8, the vacuum pump 10, the low pressure compressor 12, and the high pressure compressor 14.

The hydrogen-containing gas supplied from the first gas-liquid separator 4 to the hydrogen purification apparatus 6 is purified in the hydrogen purification apparatus 6. The hydrogen purification apparatus 6 may comprise, for example, a separation membrane through which only the hydrogen gas of the hydrogen gas and the organic compound passes selectively. The separation membrane may be, for example, a metal membrane (a PbAg-based membrane, a PdCu-based membrane, a Nb-based membrane, or the like), an inorganic membrane (a silica membrane, a zeolite membrane, a carbon membrane, or the like), or a polymer membrane (a fluororesin membrane, a polyimide membrane, or the like).

The hydrogen gas passes through the separation membrane, and thus the purity of the hydrogen gas increases. On the other hand, the organic compounds (the unreacted organic hydride and the like) in the hydrogen-containing gas cannot pass through the separation membrane. Therefore, the organic compounds are separated from the hydrogen-containing gas, and a high purity hydrogen gas is purified. The purified high purity hydrogen gas may be used as a fuel for fuel cells without going through the high pressure compressor 14 or may be compressed in the high pressure compressor 14 and then used as a fuel for fuel cells. Not only the organic compounds but also a slight amount of the hydrogen gas may not pass through the carbon membrane. The hydrogen gas that has not passed through the carbon membrane may be recovered together with the organic hydride and supplied into the dehydrogenation reactor 2 as an off-gas. Alternatively, the organic compounds that have not passed through the carbon membrane may be recovered into the tank 16. The hydrogen purification apparatus 6 is not limited to an apparatus comprising a separation membrane. The hydrogen purification apparatus 6 may be, for example, an apparatus that carries out at least one method selected from the group consisting of a pressure swing adsorption (PSA) method, a thermal swing adsorption (TSA) method (temperature swing adsorption method), a temperature pressure swing adsorption (TPSA) method, and a cryogenic separation method. It is possible to purify the hydrogen-containing gas using these apparatuses, supply into the dehydrogenation reactor 2 an off-gas produced with the purification, and supply to the tank 16 the organic compounds separated from the hydrogen-containing gas.

One aspect of the present invention has been described above, but the present invention is not limited to the above embodiments in any way.

The effects of the present invention are achieved for the first time by the combination of aluminum oxide, platinum, and a group 3 metal and are difficult to achieve without aluminum oxide. However, the carrier may contain, in addition to aluminum oxide, another component, for example, silica ($SiO_2$) or titania ($TiO_2$), in such a small amount that does not inhibit the effects of the present invention. In addition, the effects of the present invention are difficult to achieve without platinum. However, in addition to platinum, another component, for example, palladium (Pd), rhodium (Rh), iridium (Ir), or ruthenium (Ru), may be supported on the carrier in such a small amount that does not inhibit the effects of the present invention.

**Examples**

The contents of the present invention will be described in more detail below using Examples and Comparative Examples, but the present invention is not limited to the following Examples.

(Example 1)

[Step of Supporting Group 3 Metal]

As a carrier, a molded body comprising porous $\gamma$-alumina was used. The particle diameter of the molded body was about 1 to 2 mm. A scandium nitrate aqueous solution was supported on 5.18 g of this carrier by a pore filling method. Next, the carrier was dried overnight at 100°C and then calcined in air at 550°C for 3 hours to decompose scandium nitrate, and thus scandium was supported on the carrier.

[Step of Supporting Platinum]

A bis(ethanolammonium)hexahydroxoplatinic acid aqueous solution was supported by a pore filling method on the carrier on which scandium was supported. Next, the carrier was dried overnight at 100°C and then clacined in air at 330°C for 2 hours to decompose bis(ethanolammonium)hexahydroxoplatinum.

The dehydrogenation catalyst of Example 1 was made by the above steps. The dehydrogenation catalyst of Example 1 comprises a carrier comprising $\gamma$-alumina, scandium supported on the carrier, and platinum supported on the carrier. The amount of scandium supported in the dehydrogenation catalyst was 0.3% by mass based on the total mass of the carrier ($\gamma$-alumina). The amount of platinum supported in the dehydrogenation catalyst was 0.3% by mass based on the total mass of the carrier ($\gamma$-alumina).

(Example 2)

The dehydrogenation catalyst of Example 2 was made by the same method as Example 1 except that an aqueous solution of yttrium nitrate was used instead of the aqueous solution of scandium nitrate. The dehydrogenation catalyst of Example 2 comprises a carrier comprising $\gamma$-alumina, yttrium supported on the carrier, and platinum supported on the carrier. The amount of yttrium supported in the dehydrogenation catalyst was 0.4% by mass based on the total mass of the carrier ($\gamma$-alumina). The amount of platinum supported in the dehydrogenation catalyst was 0.3% by mass based on the total mass of the carrier (γ-alumina).

(Example 3)

The dehydrogenation catalyst of Example 3 was made by the same method as Example 1 except that an aqueous solution of lanthanum nitrate was used instead of the aqueous solution of scandium nitrate. The dehydrogenation catalyst of Example 3 comprises a carrier comprising γ-alumina, lanthanum supported on the carrier, and platinum supported on the carrier. The amount of La supported in the dehydrogenation catalyst was 0.5% by mass based on the total mass of the carrier (γ-alumina). The amount of platinum supported in the dehydrogenation catalyst was 0.3% by mass based on the total mass of the carrier (γ-alumina).

(Example 4)

The dehydrogenation catalyst of Example 4 was made by the same method as Example 1 except that an aqueous solution of cerium nitrate was used instead of the aqueous solution of scandium nitrate. The dehydrogenation catalyst of Example 4 comprises a carrier comprising γ-alumina, cerium supported on the carrier, and platinum supported on the carrier. The amount of cerium supported in the dehydrogenation catalyst was 0.5% by mass based on the total mass of the carrier (γ-alumina). The amount of platinum supported in the dehydrogenation catalyst was 0.3% by mass based on the total mass of the carrier (γ-alumina).

(Example 5)

The dehydrogenation catalyst of Example 5 was made by the same method as Example 4 except that the amount of cerium supported was changed in the step of supporting a group 3 metal. The dehydrogenation catalyst of Example 5 comprises a carrier comprising γ-alumina, cerium supported on the carrier, and platinum supported on the carrier. The amount of cerium supported in the dehydrogenation catalyst was 0.3% by mass based on the total mass of the carrier (γ-alumina). The amount of platinum supported in the dehydrogenation catalyst was 0.3% by mass based on the total mass of the carrier (γ-alumina).

(Example 6)

The dehydrogenation catalyst of Example 6 was made by the same method as Example 4 except that the amount of cerium supported was changed in the step of supporting a group 3 metal. The dehydrogenation catalyst of Example 6 comprises a carrier comprising γ-alumina, cerium supported on the carrier, and platinum supported on the carrier. The amount of cerium supported in the dehydrogenation catalyst was 1.0% by mass based on the total mass of the carrier (γ-alumina). The amount of platinum supported in the dehydrogenation catalyst was 0.3% by mass based on the total mass of the carrier (γ-alumina).

(Comparative Example 1)

The dehydrogenation catalyst of Comparative Example 1 was made by the same method as Example 1 except that the step of supporting a group 3 metal was not carried out. The dehydrogenation catalyst of Comparative Example 1 comprises a carrier comprising γ-alumina, and platinum supported on the carrier. The dehydrogenation catalyst of Comparative Example 1 contains no group 3 metal. The amount of platinum supported in the dehydrogenation catalyst was 0.3% by mass based on the total mass of the carrier (γ-alumina).

[Evaluation of Platinum Surface Area]

<Example 1>

The surface area of platinum contained in the dehydrogenation catalyst of Example 1 was obtained using a chemical adsorption method. In the chemical adsorption method, carbon monoxide (CO) was supplied into a container in which the dehydrogenation catalyst of Example 1 was placed. Based on the difference between the volume of CO supplied into the container and the volume of CO discharged out of the container without being adsorbed on the dehydrogenation catalyst in the container, the amount of CO adsorbed per unit mass of platinum (unit: $cm^3/g$) at 40°C was calculated. Based on this amount of CO adsorbed, the amount of platinum present on the catalyst surface and acting as an active site per unit mass of platinum, that is, the platinum surface area (unit: $m^2/g$), was calculated. The amount of CO adsorbed and the platinum surface area in Example 1 are shown in Table 1.

<Examples 2 to 6 and Comparative Example 1>

The amount of CO adsorbed and the platinum surface area in other Examples and Comparative Example 1 were obtained by the same method as Example 1. The amount of CO adsorbed and the platinum surface area in other Examples and Comparative Example 1 are shown in Table 1.

[Evaluation of Dehydrogenation Activity]

<Example 1>

A fixed-bed flow type reactor was filled with the dehydrogenation catalyst of Example 1. While methylcyclohexane was supplied into the reactor, the temperature of the central portion of the catalyst layer was maintained at 330°C, and the dehydrogenation reaction of methylcyclohexane was continued in the reactor. The liquid space velocity (LHSV) of methylcyclohexane supplied into the reactor was maintained at 11 h$^{-1}$. At a point of time when 3 hours elapsed from the start of the reaction, a gas discharged from the reactor was recovered and cooled to obtain produced oil. The produced oil was analyzed by a gas chromatograph-hydrogen flame ionization detector (GC-FID), and from the ratio between the GC area (peak area) of methylcyclohexane contained in the produced oil and the GC area of toluene contained in the liquid, the conversion rate of methylcyclohexane (unit: mol %) was calculated (see the above numerical formula (1)). The conversion rate of methylcyclohexane in Example 1 is shown in Table 1. "MCH" described in Table 1 means methylcyclohexane.

<Examples 4 to 6 and Comparative Example 1>

The conversion rate of methylcyclohexane when each of the dehydrogenation catalysts of Examples 4 to 6 and Comparative Example 1 was used alone was calculated by the same method as Example 1. The conversion rate of methylcyclohexane in Examples 4 to 6 and Comparative Example 1 is shown in Table 1. In all cases of Examples 1 and 4 to 6 and Comparative Example 1, the volume of the dehydrogenation catalyst placed in the reactor was the same.
In the evaluation of dehydrogenation activity in Examples 1 and 4 and Comparative Example 1, the conversion rate of methylcyclohexane (unit: mol %) was calculated at points of time when the elapsed time from the start of the reaction was 2 hours, 3 hours, 4 hours, and 5 hours. The conversion rate of methylcyclohexane at the points of time of the dehydrogenation reaction in Examples 1 and 4 and Comparative Example 1 is shown in Table 2.

[Table 1]

| Table I | Amount of platinum supported (% by mass) | Group 3 metal | | Amount of CO adsorbed (cm$^3$/g) | Platinum surface area (m$^2$/g) | Conversion rate of MCH (mol%) |
|---|---|---|---|---|---|---|
| | | | Amount supported (% by mass) | | | |
| Example 1 | 0.3 | Sc | 0.3 | 0.809 | 199.9 | 78.7 |
| Example 2 | 0.3 | Y | 0.4 | 0.753 | 186.0 | - |
| Example 3 | 0.3 | La | 0.5 | 0.696 | 172.0 | - |
| Example 4 | 0.3 | Ce | 0.5 | 0.694 | 1714 | 81.9 |
| Example 5 | 0.3 | Ce | 0.3 | 0.718 | 177.4 | 75.8 |
| Example 6 | 0.3 | Ce | 1.0 | 0.753 | 186.0 | 79.4 |
| Comparative Example 1 | 0.3 | - | 0.0 | 0.537 | 130.2 | 75.2 |

[Table 2]

| Table 2 | Group 3 metal | Elapsed time | | | |
|---|---|---|---|---|---|
| | | 2 hours | 3 hours | 4 hours | 5 hours |
| Example 1 | Sc | 80.8 mol% | 78.7 mol% | 76.1 mol% | 74.3 mol% |

(continued)

| Table 2 | Group 3 metal | Elapsed time | | | |
|---|---|---|---|---|---|
| | | 2 hours | 3 hours | 4 hours | 5 hours |
| Example 4 | Ce | 83.6 mol% | 81.9 mol% | 80.3 mol% | 78.7 mol% |
| Comparative Example 1 | - | 77.8 mol% | 75.2 mol% | 74.0 mol% | 71.4 mol% |

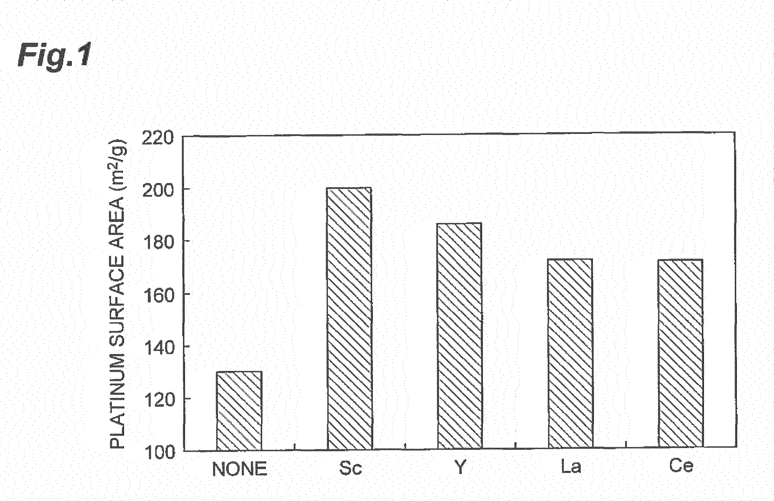

The platinum surface area in each of the dehydrogenation catalysts of Examples 1 to 4 and Comparative Example 1 is shown in Figure 1. In Figure 1, "NONE" corresponds to Comparative Example 1. "Sc" corresponds to Example 1. "Y" corresponds to Example 2. "La" corresponds to Example 3. "Ce" corresponds to Example 4. Table 1 and Figure 1 show that the platinum surface area increases by adding a group 3 metal to $\gamma$-alumina supporting platinum.

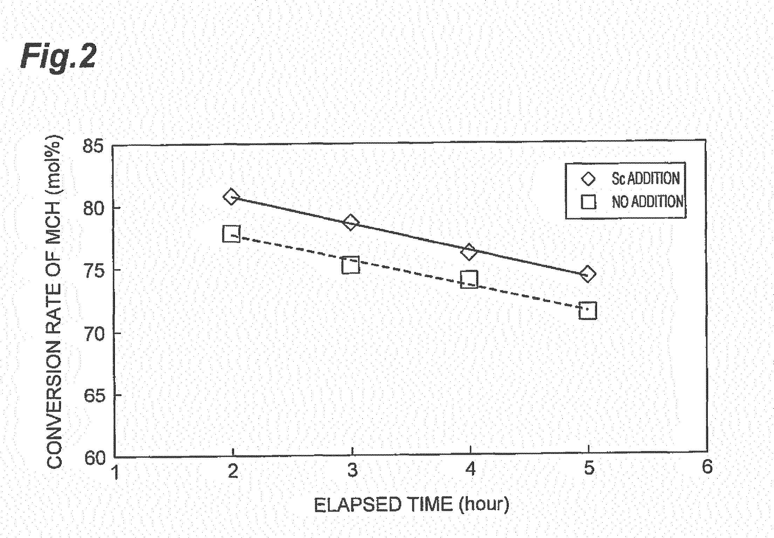

The conversion rate of methylcyclohexane at the points of time of the dehydrogenation reaction in Example 1 and Comparative Example 1 is shown in Figure 2. In Figure 2, "NO ADDITION" corresponds to Comparative Example 1. "Sc ADDITION" corresponds to Example 1. Table 2 and Figure 2 show that the dehydrogenation activity improves by adding scandium to $\gamma$-alumina supporting platinum.

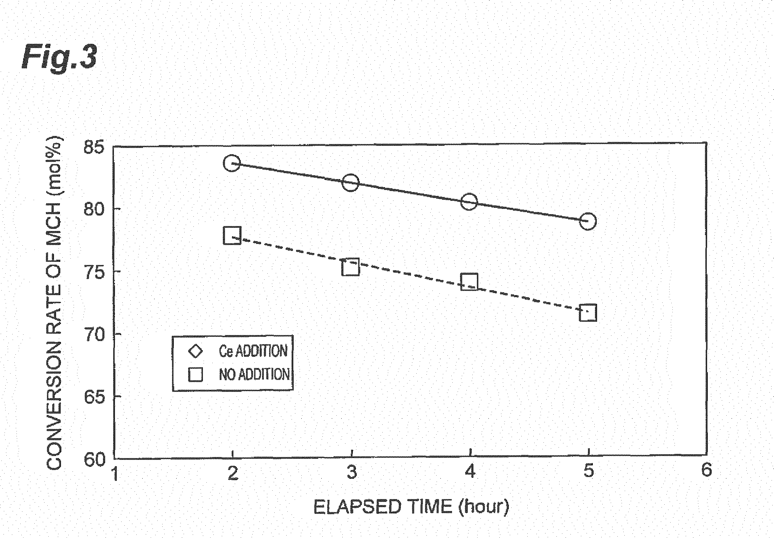

The conversion rate of methylcyclohexane at the points of time of the dehydrogenation reaction in Example 4 and Comparative Example 1 is shown in Figure 3. In Figure 3, "NO ADDITION" corresponds to Comparative Example 1. "Ce ADDITION" corresponds to Example 4. Table 2 and Figure 3 show that the dehydrogenation activity improves by adding cerium to $\gamma$-alumina supporting platinum.

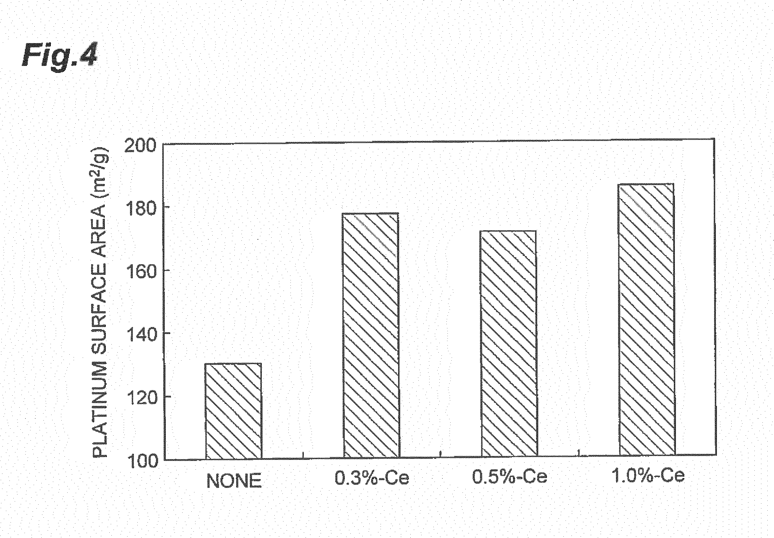

The platinum surface area in each of the dehydrogenation catalysts of Examples 4 to 6 and Comparative Example 1 is shown in Figure 4. In Figure 4, "NONE" corresponds to Comparative Example 1. "0.3%-Ce" corresponds to Example 5. "0.5%-Ce" corresponds to Example 4. "1.0%-Ce" corresponds to Example 6. Table 1 and Figure 4 show that in a range in which the amount of cerium added is 0.3 to 1.0%, the platinum surface area increases by the addition of cerium regardless of the amount of cerium added based on $\gamma$-alumina supporting platinum.

(Examples 11 to 15)

Predetermined amounts of water, an aqueous solution of cerium nitrate, and dilute nitric acid were added to a powder of a hydroxide of aluminum in a pseudoboehmite state, and these were kneaded. The pH of the kneaded material was adjusted to about 3 to 7 by the addition of dilute nitric acid. Pellets were made by the extrusion of the kneaded material. The pellets were dried at 100 to 150°C for 2 hours and then calcined at 550°C for 2 hours to make pellets comprising $\gamma$-alumina on which $C_2O_3$ was supported. An aqueous solution of ethanolamineplatinum was supported on the pellets after the calcining, and then the pellets were dried. The pellets after the drying were calcined at 330°C for 2 hours.

The dehydrogenation catalysts of Examples 11 to 15 comprising $\gamma$-alumina (carrier), platinum, and cerium oxide ($Ce_2O_3$) were made by the above steps. The amount of platinum supported in each of the dehydrogenation catalysts of Examples 11 to 15 was adjusted to 0.3% by mass in terms of a platinum elemental substance based on the total mass of $\gamma$-alumina. The amount of $Ce_2O_3$ supported in each of the dehydrogenation catalysts of Examples 11 to 15 was adjusted to a value shown in the following Table 3. The amount of $Ce_2O_3$ supported shown in the following Table 3 is a ratio to the total mass of $\gamma$-alumina.

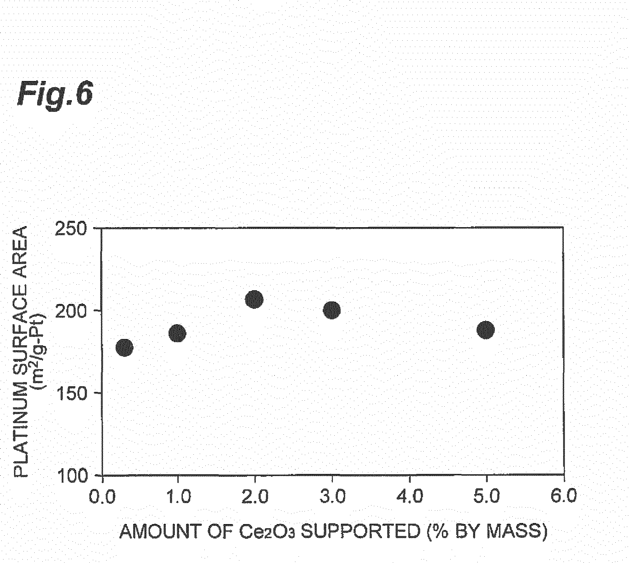

The platinum surface area in each of the dehydrogenation catalysts of Examples 11 to 15 was obtained by the same method as Example 1. The platinum surface area in each Example is shown in the following Table 3. The relationship between the amount of $Ce_2O_3$ supported and the platinum surface area in each of the dehydrogenation catalysts of Examples 11 to 15 is shown in Figure 6.

[Table 3]

| Table 3 | Amount of $Ce_2O_3$ supported (% by mass) | Platinum surface area ($m^2/g$) |
|---|---|---|
| Example 11 | 2.0 | 206.7 |
| Example 12 | 3.0 | 200.0 |
| Example 13 | 5.0 | 187.9 |
| Example 14 | 0.3 | 177.4 |
| Example 15 | 1.0 | 186.0 |

As shown in Table 3 and Figure 6, it was confirmed that when the amount of $Ce_2O_3$ supported was 2.0 to 3.0% by mass, the platinum surface area was particularly large.

(Examples 21 to 24)

In the making of the dehydrogenation catalysts of Examples 21 to 24, the amount of platinum supported based on the total mass of $\gamma$-alumina was adjusted to a value shown in the following Table 4, and the amount of $Ce_2O_3$ supported based on the total mass of $\gamma$-alumina was adjusted to 2.0% by mass. The dehydrogenation catalysts of Examples 21 to 24 were made by the same method as Examples 11 to 15 except these matters. All of the dehydrogenation catalysts of Examples 21 to 24 comprised $\gamma$-alumina (carrier), platinum, and cerium oxide ($Ce_2O_3$). The platinum dispersion degree in each of the dehydrogenation catalysts of Examples 21 to 24 was obtained based on the following formula 2. The platinum dispersion degree in each Example is shown in the following Table 4.

$$Dm = V_{chem} \times (SF/22414) \times Mw \times (1/c) \times 100 \quad (2)$$

Dm is the platinum dispersion degree (unit: %). $V_{chem}$ is the amount of CO adsorbed (unit: $cm^3$) in the dehydrogenation catalyst. SF is the stoichiometric ratio of CO adsorption and is 1. Mw is the atomic weight of platinum (unit: g/mol).

The dehydrogenation reaction of methylcyclohexane using each of the dehydrogenation catalysts of Examples 21 to 24 was performed at five reaction temperatures, 270°C, 280°C, 290°C, 300°C, and 310°C. The dehydrogenation reaction using each of the dehydrogenation catalysts of Examples 21 to 24 was performed by the same method as Example 1 except the reaction temperatures. An Arrhenius plot (lnk vs. 1/T) was made based on the following formula 3:

$$lnk = ln[SV \times ln\{1/(1 - conv.)\}]$$
$$= lnA - E/RT \quad (3)$$

ln is the natural logarithm. k is the rate constant of the dehydration reaction. T is each reaction temperature. conv. is the conversion rate of methylcyclohexane in the dehydrogenation reaction at each reaction temperature T. SV is the liquid space velocity of methylcyclohexane supplied into the reactor in each dehydrogenation reaction.

Next, the frequency factor A was obtained from the intercept of the Arrhenius plot, and the rate constant $k_{300}$ when the reaction temperature was 300 K was obtained from the Arrhenius formula. The rate constant $k_{300}$ is represented by the following formula 4:

$$k_{300} = A \times e^{-E/RT} \quad (4)$$

Next, the reaction rate $r_{300}$ at 300°C was obtained by normalization based on the amount of platinum and the amount of passing oil. The reaction rate $r_{300}$ is represented by the following formula 5:

$$r_{300} = -k_{300} \times \{(1 - conv.)/w(Pt)\} \quad (5)$$

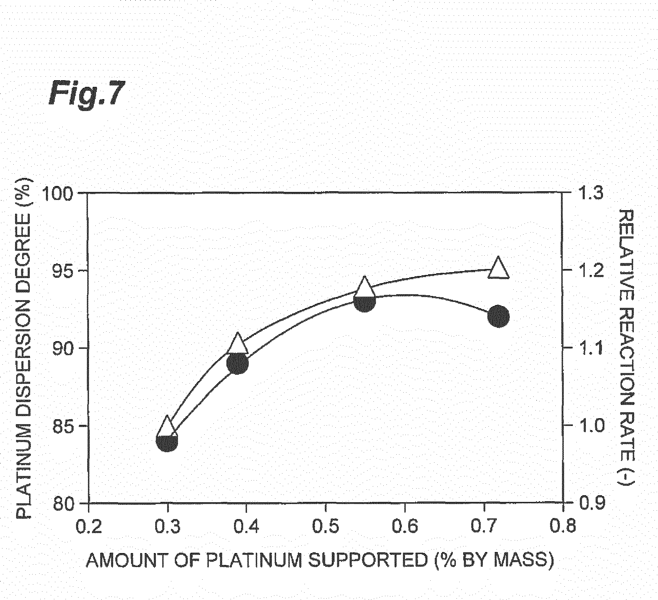

By dividing the reaction rate $r_{300}$ of the dehydrogenation reaction of methylcyclohexane using each dehydrogenation catalyst by the reaction rate $r_{300}$ when the dehydrogenation catalyst of Example 21 was used, the relative reaction rate of the dehydrogenation reaction of methylcyclohexane using each dehydrogenation catalyst was obtained. The relative reaction rate when each dehydrogenation catalyst is used is shown in the following Table 4. In addition, the ratio $m_3/m_P$ of the amount of platinum supported ($m_P$% by mass) and the amount of $Ce_2O_3$ supported ($m_3$% by mass) in each dehydrogenation catalyst is shown in the following Table 4. The platinum dispersion degree of each dehydrogenation catalyst, and the relative reaction rate when each dehydrogenation catalyst is used are shown in Figure 7. A circle mark in Figure 7 represents a platinum dispersion degree, and a triangle mark represents a relative reaction rate.

[Table 4]

| Table 4 | Amount of platinum supported (% by mass) | $m_3/m_P$ (-) | Platinum dispersion degree (%) | Relative reaction rate (-) |
|---|---|---|---|---|
| Example 21 | 0.30 | 6.67 | 84 | 1.00 |
| Example 22 | 0.39 | 5.13 | 89 | 1.11 |
| Example 23 | 0.55 | 3.64 | 93 | 1.18 |
| Example 24 | 0.72 | 2.78 | 92 | 1.20 |

As shown in Table 4 and Figure 7, it was found that when the amount of platinum supported was about 0.5 to 0.6% by mass (when $m_3/m_P$ was (10/3) to 4), the platinum functioned efficiently.

(Examples 31 to 37 and Comparative Example 31)

In the making of the dehydrogenation catalysts of Examples 31 to 37, the water absorption rate of a commercial $\gamma$-alumina carrier was measured. Based on this water absorption rate, an aqueous solution containing a nitrate of a group 3 metal shown in the following Table 5 at a predetermined concentration was prepared, and the aqueous solution was supported on $\gamma$-alumina. Next, the carrier was dried at 100°C for 8 hours and then calcined at 500°C for 2 hours. An aqueous solution of a platinum compound was supported on the carrier after the calcining. Next, the carrier was dried, and calcined at 330°C for 2 hours. The dehydrogenation catalysts of Examples 31 to 37 were made by the above steps.

The amount of the oxide of the group 3 metal supported in each dehydrogenation catalyst was adjusted to a value shown in the following Table 5. The amount of platinum supported in each dehydrogenation catalyst was adjusted to a value shown in the following Table 5. The number of moles of the oxide of the group 3 metal and the number of moles of platinum in each dehydrogenation catalyst were values shown in the following Table 5. The ratio of the number of moles of the oxide of the group 3 metal to the number of moles of platinum (Metal/Pt) in each dehydrogenation catalyst was a value shown in the following Table 5.

In the making of the dehydrogenation catalyst of Comparative Example 31, the step of supporting an aqueous solution of a nitrate of a group 3 metal on $\gamma$-alumina was not carried out, and only platinum was supported on $\gamma$-alumina. The amount of platinum supported in the dehydrogenation catalyst of Comparative Example 31 was adjusted to a value shown in the following Table 5. The number of moles of platinum in the dehydrogenation catalyst of Comparative Example 31 was a value shown in the following Table 5.

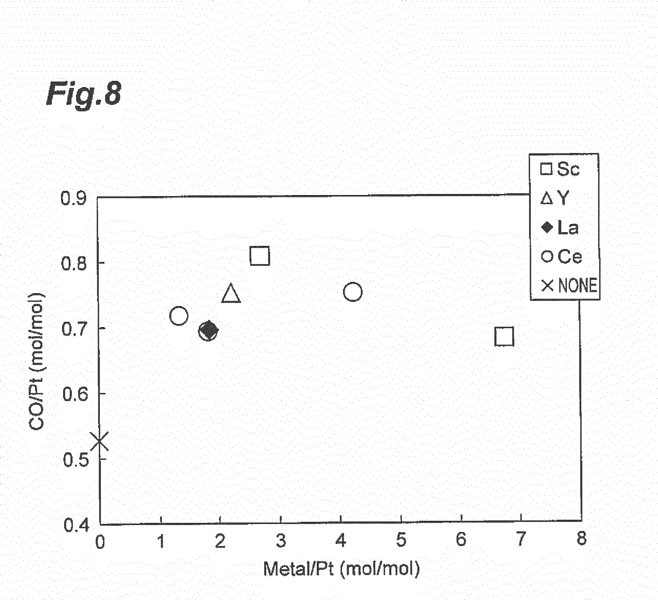

The ratio of the number of moles of CO adsorbed on platinum to the number of moles of platinum (CO/Pt) in each dehydrogenation catalyst was obtained using the same chemical adsorption method as Example 1. CO/Pt in each dehydrogenation catalyst is shown in the following Table 5. The relationship between Metal/Pt and CO/Pt in each dehydrogenation catalyst is shown in Figure 8.

[Table 5]

| Table 5 | Group 3 metal | Amount of oxide of group 3 metal supported | | Amount of platinum supported | | Metal/Pt | CO/Pt |
|---|---|---|---|---|---|---|---|
| | | (% by mass) | (mol) | (g) | (mol) | (mol/mol) | (mol/mol) |
| Example 31 | Sc | 0.3 | 0.000228 | 0.016 | 0.0000842551 | 2.7 | 0.81 |
| Example 32 | Sc | 0.5 | 0.001112 | 0.032 | 0.0001647914 | 6.75 | 0.68 |
| Example 33 | Y | 0.4 | 0.000198 | 0.017 | 0.0000890787 | 2.22 | 0.75 |
| Example 34 | La | 0.5 | 0.000168 | 0.018 | 0.0000907971 | 1.85 | 0.7 |
| Example 35 | Ce | 0.3 | 0.000216 | 0.031 | 0.0001599394 | 1.35 | 0.72 |
| Example 36 | Ce | 0.5 | 0.000162 | 0.017 | 0.0000886910 | 1.83 | 0.69 |
| Example 37 | Ce | 1 | 0.000836 | 0.038 | 0.0001970324 | 4.24 | 0.75 |
| Comparative Example 31 | None | 0 | 0 | 0.030 | 0.000153798 | 0 | 0.53 |

It was confirmed that in the dehydrogenation catalysts of Examples 31 to 37 comprising an oxide of a group 3 metal, CO/Pt was large, and the platinum specific surface area was large, compared with Comparative Example 31.

(Examples 41 to 45)

In the making of the dehydrogenation catalysts of Examples 41 to 45, predetermined amounts of water, an aqueous solution of cerium nitrate, and dilute nitric acid were added to a powder of a hydroxide of aluminum in a pseudoboehmite state, and these were kneaded. The pH of the kneaded material was adjusted to about 3 to 7 by the addition of dilute nitric acid. Pellets were made by the extrusion of the kneaded material. The pellets were dried at 100 to 150°C for 2 hours and then calcined at 550°C for 2 hours to make pellets comprising γ-alumina on which $C_2O_3$ was supported. An aqueous solution of a platinum compound shown in the following Table 6 was supported on the pellets after the calcining, and then the pellets were dried. The pellets after the drying were calcined at 330°C for 2 hours.
The dehydrogenation catalysts of Examples 41 to 45 comprising γ-alumina (carrier), platinum, and cerium oxide ($Ce_2O_3$) were made by the above steps. It was confirmed that each of the dehydrogenation catalysts of Examples 41 to 45 shows shading of brown. The amount of $Ce_2O_3$ supported in each dehydrogenation catalyst was adjusted to 2% by mass based on the total mass of γ-alumina. The concentration of the aqueous solution of each of the platinum compounds used in Examples 41 to 45 was adjusted to a value shown in the following Table 6. The pH of the aqueous solution of each of the platinum compounds used in Examples 41 to 45 was adjusted to a value shown in the following Table 6. The valence of platinum in each of the platinum compounds used in Examples 41 to 45 was a value shown in the following Table 6. The platinum surface area in each of the dehydrogenation catalysts of Examples 41 to 45 was obtained by the same method as Example 1. The platinum surface area in each dehydrogenation catalyst of Examples 41 to 45 is shown in the following Table 6.

[Table 6]

| Table 6 | Platinum compound | Concentration (% by mass) | pH (-) | Valence of platinum (-) | Platinum surface area ($m^2$/g) |
|---|---|---|---|---|---|
| Example 41 | Dinitrodiammine platinum nitrate | 10 | 0 | 2 | 201 |
| Example 42 | Ethanolamine platinum | 10 | 10 | 4 | 184 |
| Example 43 | Hexaammine platinum hydroxy salt | 1 | 13 | 4 | 164 |
| Example 44 | Platinum nitrate | 10 | 0 | 2 | 116 |
| Example 45 | Dinitrodiammine platinum ammonia solution | 3 | 11 | 2 | 206 |

As shown in Table 6, it was confirmed that the platinum surface area in Examples 41 to 43 and 45 was large compared with Example 44.

Industrial Applicability

A hydrogen gas obtained by the dehydrogenation reaction of a naphthenic hydrocarbon using the dehydrogenation catalyst according to the present invention is used, for example, for a fuel for fuel cells.

Reference Signs List

2 ... dehydrogenation reactor, 4 ... first gas-liquid separator, 6 ... hydrogen purification apparatus, 8 ... second gas-liquid separator (degassing apparatus), 10 ... vacuum pump, 12 ... low pressure compressor, 14 ... high pressure compressor, 16 ... tank, 100 ... system for producing hydrogen.

**Claims**

1. A dehydrogenation catalyst for naphthenic hydrocarbons comprising:

a carrier containing aluminum oxide;
platinum; and
a group 3 metal.

2.  The dehydrogenation catalyst according to claim 1, wherein
    an amount of the group 3 metal supported is 0.1 to 5.0% by mass in terms of an oxide of the group 3 metal based on a total mass of the aluminum oxide.

3.  The dehydrogenation catalyst according to claim 1 or 2, wherein
    when an amount of the platinum supported is $m_P$% by mass in terms of a platinum elemental substance based on the total mass of the aluminum oxide, and
    the amount of the group 3 metal supported is $m_3$% by mass in terms of the oxide of the group 3 metal based on the total mass of the aluminum oxide,
    $m_3/m_P$ is (10/3) to 4.

4.  A method for producing a dehydrogenation catalyst for naphthenic hydrocarbons, comprising:

    a step of making a carrier which contains aluminum oxide and on which a group 3 metal is supported; and
    a step of supporting a solution of a platinum compound on the carrier and calcining the carrier.

5.  The method for producing a dehydrogenation catalyst according to claim 4, wherein
    the platinum compound contains an amine or ammonia.

6.  The method for producing a dehydrogenation catalyst according to claim 4 or 5, wherein
    the carrier is made by kneading a hydroxide of aluminum in a pseudoboehmite state, an aqueous solution of a nitrate of the group 3 metal, and nitric acid to prepare a kneaded material, making a pellet by extrusion of the kneaded material, and calcining the pellet.

7.  A system for producing hydrogen, comprising a dehydrogenation reactor having the dehydrogenation catalyst according to any one of claims 1 to 3 and producing hydrogen by dehydrogenation of a naphthenic hydrocarbon using the dehydrogenation catalyst.

8.  A method for producing hydrogen, comprising a step of producing hydrogen by dehydrogenation of a naphthenic hydrocarbon using the dehydrogenation catalyst according to any one of claims 1 to 3.

*Fig.1*

EP 2 979 756 A1

Fig.2

*Fig.3*

Fig.4

Fig.5

SYSTEM FOR PRODUCING HYDROGEN 100

ORGANIC HYDRIDE

OFF-GAS

HYDROGEN GAS

HYDROGEN GAS

HYDROGEN GAS

HYDROGEN PURIFICATION APPARATUS 6

HIGH PRESSURE COMPRESSOR 14

GAS PHASE

DEHYDROGENATION REACTOR 2

LOW PRESSURE COMPRESSOR 12

HYDROGEN GAS

FIRST GAS-LIQUID SEPARATOR 4

VACUUM PUMP 10

ORGANIC COMPOUND (LIQUID)

LIQUID PHASE

SECOND GAS-LIQUID SEPARATOR (DEGASSING APPARATUS 8)

TANK 16

*Fig.6*

Fig.7

Fig.8

## INTERNATIONAL SEARCH REPORT

| International application No. |
| --- |
| PCT/JP2014/058281 |

A. CLASSIFICATION OF SUBJECT MATTER
*B01J23/63*(2006.01)i, *C01B3/02*(2006.01)i, *H01M8/06*(2006.01)i

According to International Patent Classification (IPC) or to both national classification and IPC

B. FIELDS SEARCHED

Minimum documentation searched (classification system followed by classification symbols)
B01J23/63, C01B3/02, H01M8/06

Documentation searched other than minimum documentation to the extent that such documents are included in the fields searched
Jitsuyo Shinan Koho          1922–1996   Jitsuyo Shinan Toroku Koho   1996–2014
Kokai Jitsuyo Shinan Koho    1971–2014   Toroku Jitsuyo Shinan Koho   1994–2014

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X Y | JP 11-507685 A  (Institut Francais du Petrole), 06 July 1999 (06.07.1999), claims 1, 7, 26; page 16, line 26 to page 17, line 6; example 15; table 6 & US 6045689 A        & EP 832170 A & WO 1997/000306 A1      & FR 2735490 A & FR 2735491 A          & FR 2735492 A & FR 2735493 A          & FR 2735490 A1 & ES 2163030 T          & RU 2157826 C & CA 2225498 A          & CN 1187843 A | 1-5,7,8 6 |
| Y | JP 2007-69151 A  (JGC Corp.), 22 March 2007 (22.03.2007), paragraphs [0021], [0046] & US 2008/0224097 A1     & EP 1930076 A1 & WO 2007/029862 A1      & ZA 200802963 A & BRA PI0615450          & AU 2006288168 A | 6 |

| ☒ | Further documents are listed in the continuation of Box C. | ☐ | See patent family annex. |

\* Special categories of cited documents:
"A" document defining the general state of the art which is not considered   to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 10 June, 2014 (10.06.14) | 17 June, 2014 (17.06.14) |

| Name and mailing address of the ISA/ Japanese Patent Office | Authorized officer |
| --- | --- |
| Facsimile No. | Telephone No. |

Form PCT/ISA/210 (second sheet) (July 2009)

**INTERNATIONAL SEARCH REPORT**

International application No.

PCT/JP2014/058281

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2009-125699 A  (JGC Catalysts and Chemicals Ltd.), 11 June 2009 (11.06.2009), paragraphs [0023], [0024], [0028] & US 2009/0036299 A1    & TW 200927283 A | 6 |
| A | JP 2012-210620 A  (JX Nippon Oil & Energy Corp.), 01 November 2012 (01.11.2012), claims 1, 4; paragraph [0066]; examples 3, 8, 9 (Family: none) | 1-8 |
| A | JP 2011-88066 A  (JX Nippon Oil & Energy Corp.), 06 May 2011 (06.05.2011), claims 1, 2; paragraphs [0001], [0016], [0017], [0021] (Family: none) | 1-8 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **R. W. COUGHLIN ; K. KAWAKAMI ; AKRAM HASAN.** *Journal of Catalysis,* 1984, vol. 88, 150-162